(19) Europäisches Patentamt
European Patent Office
Office européen des brevets

(11) Publication number: **0 102 086**
**A2**

(12) # EUROPEAN PATENT APPLICATION

(21) Application number: 83108544.4

(22) Date of filing: 30.08.83

(51) Int. Cl.³: C 07 C 121/40
C 07 C 121/66, C 07 C 121/76
C 07 C 121/00

(30) Priority: 31.08.82 JP 151003/82
31.08.82 JP 151004/82

(43) Date of publication of application:
07.03.84 Bulletin 84/10

(84) Designated Contracting States:
CH DE FR GB LI

(71) Applicant: Daikin Kogyo Co., Ltd.
Shinhankyu Building No 1-12-39, Umeda Kita-ku
Osaka-shi Osaka-fu(JP)

(72) Inventor: Ishikawa, Nobuo, Dr.
10-10, Shinoharadai-machi Kohoku-ku
Yokohama-shi Kanagawa-ken(JP)

(72) Inventor: Takaoka, Akio, Dr.
17-109, Dobashi 1-chome Takatsu-ku
Kawasaki-shi Kanagawa-ken(JP)

(74) Representative: Patentanwälte TER MEER - MÜLLER - STEINMEISTER
Triftstrasse 4
D-8000 München 22(DE)

(54) **Alpha-substituted-alpha-fluoro-alpha-cyanoacetic acid or its derivatives.**

(57) An α-substituted-α-fluoro-α-cyanoacetic acid or its derivatives that are expressed by a general formula:

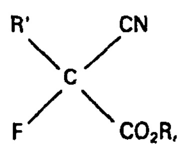

where R is an aliphatic or aromatic hydrocarbon group, or hydrogen or alkali metal atom and R' is an aliphatic or aromatic hydrocarbon group or a group that derived from an acceptor of the Michael reaction.

EP 0 102 086 A2

# SPECIFICATION

## BACKGROUND OF THE INVENTION

### Title of the Invention

An α-substituted-α-fluoro-α-cyanoacetic acid and its derivatives.

### Field of the Invention

This invention relates to an α-substituted-α-fluoro-α-cyanoacetic acid and its derivatives.

### Description of the Prior Art

There are known many monofluoro compounds that have physiological activities and/or are efficacious as agricultural chemicals or medicines. As a building block of such monofluoro compounds, the α-fluoro-α-cyanoacetic acid ester is thus considered to be a very important intermediate.

## OBJECTS AND SUMMARY OF THE INVENTION

Accordingly, it is an object of the present invention to provide a new and useful fluorine containing cyanoacetic acid or its derivatives.

Namely, the invention provides an α-substituted-α-fluoro-α-cyanoacetic acid or its derivatives that are characteristically expressed by the following general formula:

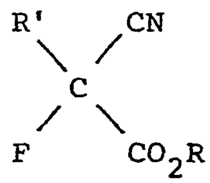 ,

where R is an aliphatic or aromatic hydrocarbon group, or hydrogen or alkali metal atom while R' an aliphatic or aromatic hydrocarbon group or a group that derived from an acceptor of the Michael reaction.

The above cyanoacetic acid and its derivatives of the invention have a fluorine atom in their molecular structure and therefore exhibit superior properties of the fluoro compound while their cyano group gives them an efficacy as agricultural chemicals such as the herbicide.

R and R' in the above general formula may be same or different groups selected from among alkyl groups having up to 10 carbon atoms, for example, methyl, ethyl, propyl and butyl groups, and allyl and other alkenyl groups, aryl and other unsubstituted and substituted aromatic hydrocarbon groups.

Further, the above group R' is preferably a group that derives from an acceptor of the Michael reaction, for example, an unsaturated compound expressed by the following general formula:

YCH=CHR" or YC≡CR",

where R" is a hydrogen atom or aliphatic group preferably having carbon atoms of 1 to 6, and Y is a highly electronegative group with or without formation of a ring with the carbon atom to which R" is bonded.

Y is preferably an alkylcarbonyl or alkoxycarbonyl group preferably having carbon atoms of 1 to 6, or cyano group bonded to a carbon atom comprising the unsaturated carbon-carbon bond.

DESCRIPTION OF THE PREFERRED EMBODIMENTS

Examples of the compounds of the present invention will be concretely described in terms of their manufacturing method.

For example, using hexafluoropropene (HFP) as the starting material, the following reaction is conducted to prepare ethyl α-fluoro-α-cyanoacetate 2 through tetrafluoropropionitrile 1 with ethyl monofluoromalonate 3 as by-product:

$$\underset{\substack{\text{HFP} \\ \text{(Bubbling)}}}{CF_3CF=CF_2} \xrightarrow[\text{0-10°C}]{NH_3\text{ aq./Dioxane}} \underset{\substack{\underline{1} \\ \text{(Yield: 75-80\%)}}}{CF_3CHFCN}$$

$$\underline{1} \xrightarrow[\text{2) } H_3O^+]{\text{1) } NaOH/C_2H_5OH} NCCHFCO_2C_2H_5$$

$\underline{2}$

(Yield: 60%)

+

$C_2H_5O_2CCHFCO_2C_2H_5$

(Yield: 25%)

In the above reaction, the nitrile $\underline{1}$ is produced in a short reaction time with a much improvement in yield by using a dry ice-acetone condenser and having HFP bubble into ammonia-dioxane solution at a controlled reaction temperature. The above synthesis of compound $\underline{1}$ is conducted by a method as disclosed by Knunyants and others wherein ammonia, if used in an excessive amount and particularly more than several times its stoichiometric quantity, much improves the yield to 75 to 80% as mentioned above.

Next, this nitrile $\underline{1}$ is reacted with an alkoxide or alcoholic potassium hydroxide solution for an alcoholysis. The reaction mixture is then acidified by addition of hydrochloric acid or the like for selective formation of the above malonic ester $\underline{3}$.
If, after the alcoholysis under the action of an alcoholic alkali hydroxide solution, for example, ethanolic or propanolic sodium hydroxide solution, the alcohol is removed and hydrochloric acid is then added for

acidification, the product 2 will be produced almost in a yield of 70%.

Since the products 2 and 3 have boiling points that differ by 35°c, it is possible to isolate and purify the compound 2 by rectification (yield of 2: 60%, boiling point: 174-175°C).

A possible reaction scheme for the mechanism by which the compound 2 is formed from 1 is given below, in which the reaction proceeds through formation of perfluoroacrylonitrile as an intermediate. It is assumed that ethyl fluorocyanoorthoacetate 4 is then formed. If it is hydrolyzed by an acid, such as HCl, $H_2SO_4$ or the like, there is produced the compound 2. Further, partially, the hydrochloride of ethyl α-fluorocarboethoxyimidoacetate is formed and, as its cyano group is decomposed, the compound 3 is produced.

$$\underset{\underline{1}}{CF_3CHFCN} \xrightarrow{OH^{\ominus}} [CF_3\overset{\ominus}{C}FCN]$$

$$\underset{}{\overset{-F}{\rightleftharpoons}} [CF_2{=}CFCN]$$

$$\xrightarrow{C_2H_5OH} [C_2H_5OCF_2CHFCN]$$

$$\xrightarrow{OH^{\ominus}} [C_2H_5OCF_2\overset{\ominus}{C}FCN]$$

$$\xrightarrow[C_2H_5OH]{-F^{\ominus}} \underset{\underline{4}}{(C_2H_5O)_3CCHFCN}$$

$$\xrightarrow{H_3O^+} C_2H_5O_2CCHFCN$$

2

+

$$C_2H_5O_2CCHFCO_2C_2H_5$$

3

It is noted that the ester 2 produced from the above reaction can be hydrolyzed to α-fluoro -α-cyanoacetic acid 5 or treated with alkali hydroxide to form an alkali salt 6, for example, sodium salt of α-fluoro-α-cyanoacetic acid according to the following reaction:

$$C_2H_5O_2CCHFCN \xrightarrow{H^+} HO_2CCHFCN + C_2H_5OH$$

5

or

$$C_2H_5O_2CCHFCN \xrightarrow{NaOH} NaO_2CCHFCN + C_2H_5OH$$

6

In the above method of manufacturing the compound 2, HFP industrially produced is used as the starting material and it is reacted with ammonia and the resultant compound is particularly subjected to an alcoholysis reaction followed by a treatment with a mineral acid. So the α-fluoro-α-cyanoacetate ester can be produced at a low cost under a harmless condition.

The alkyl (ethyl) group in the ethoxycarbonyl group of

the above ester 2 derives from the alkoxide or alcohol used in the alcoholysis as mentioned above.

If the hydrocarbon group of such alkoxide or alcohol is selected so as to correspond to the given R, therefore, the alkylated or alkenylated ester of the invention can be produced as mentioned below.

For example, the above ethyl α-fluoro-α-cyanoacetate can be alkylated or alkenylated under presence of various bases at room temperature. Since, among these bases, NaH performs excellently, first, the ester is added to ice-cooled NaH in diglyme to form a carbanion from the compound 2 , then, an alkyl or alkenyl halide R'X where R' is particularly an aliphatic hydrocarbon group having up to 10 carbon atoms and X is a halogen atom is added dropwise for agitation at room temperature. The above reaction of alkylation or alkenylation proceeds according to the following formula, which gives the alkylated or alkenylated products as expressed by the chemical formula 7 in high yield as in Table 1:

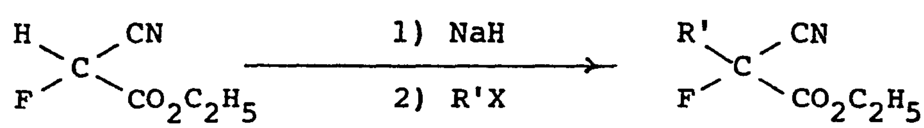


2 7

Table 1

| R'X | Reaction time, hr | R' of product 7 | $^{19}$FNMR Yield, % | $^{19}$FNMR δ(ppm) | $J_{HF}$ ($H_2$) |
|---|---|---|---|---|---|
| $CH_3Br$ | 2 | $CH_3$- | 86 | 71.0 (q) | 21.8 |
| $C_2H_5Br$ | 5 | $C_2H_5$- | 73 | 78.5 (t) | 20.7 |
| $CH_2=CHCH_2Br$ | 5 | $CH_2=CHCH_2$- | 71 | 81.0 (t) | 21.4 |
| $n-C_4H_9Br$ | 5 | $n-C_4H_9$- | 74 | 114.6 (t) | 21.6 |

Having a cyano group, such alkylated or alkenylated α-fluoro-α-cyanoacetate ester 7 is superbly efficacious as agricultural chemicals, for example, as a herbicide. In addition, the above compounds 2 and 7 can be used to synthesize derivatives of pyrimidine.

On the other hand, the above compound 2 is subjected to a 10 min reaction under agitation and under presence of a catalytic amount of spray-dried KF in sulfolane at room temperature to produce the following carbanion 8:

$NCCHFCO_2C_2H_5$

2

$$\xrightarrow{\text{Base}} [NCC^{\ominus}FCOO\text{-}C_2H_5]$$

8

Various acceptors of Michael reaction or Michael acceptors can then be added to the above carbanion in an ice -cooled condition to give a reaction as expressed by the following formula at room temperature for immediate

conversion to the corresponding Michael adducts at high yield:

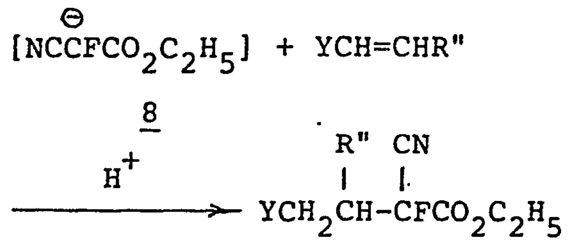


8

9

where YCH=CHR" is a Michael acceptor, R" being a hydrogen atom or alkyl group and Y a highly electronegative group, for example, alkylcarbonyl, alkoxycarbonyl or cyano group. Y may form a ring by bonding to the carbon atom to which R" is bonded. The above Michael reaction can also use an acceptor that has a carbon-carbon triple bond. In this case, the reaction proceeds as follows:

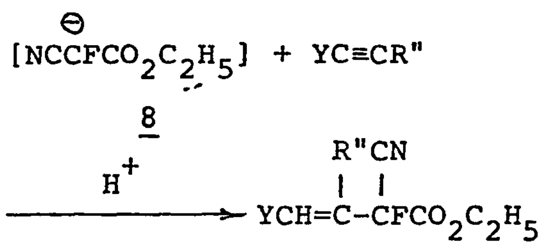


8

9

The following Table 2 gives several examples of the Michael adduct 9 as derived from the ethyl α-fluoro-α-cyanoacetate by the above method.

Table 2

| Michael acceptor | Reaction time | Product (Michael adduct) | $^{19}F$ NMR Yield, % | $^{19}F$ NMR δ(ppm) | $^{19}F$ NMR $J_{HF}$ ($H_2$) |
|---|---|---|---|---|---|
| 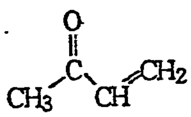 | 10 min | 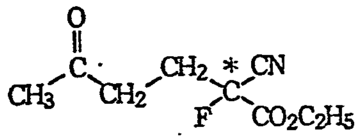 | 79 | 77.3 (t) | 20.9 |
| 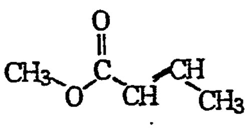 | 2 hr | 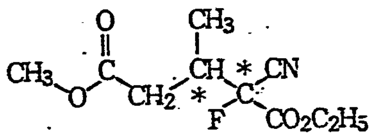 | 95 | 81.3 (d)<br>82.8 (d) | 16.9 |
| 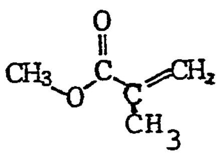 | 2 hr | 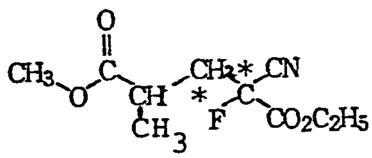 | 94 | 76.5 (dd)<br>78.8 (dd) | 19.8<br>21.6 |
| 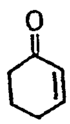 | 10 min | 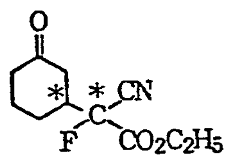 | 99 | 83.5 (d)<br>84.5 (d) | 17.9 |
| 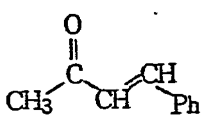 | 10 min | 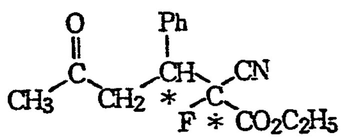 | 93 | 78.9 (d)<br>81.4 (d) | 22.0<br>22.9 |
| 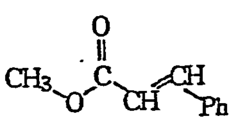 | 15 hr | 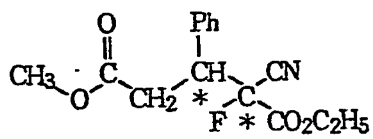 | 92 | 79.5 (d)<br>83.5 (d) | 20.7<br>23.5 |

These Michael adducts can be used as agricaltural chemicals, for example, herbicide, insecticide or these intermediates

The above embodiments are set forth as a further description but are not to be construed as limiting the invention thereto. Modifications and variations are

possible without departing from the spirit and scope of the invention.

For example, if alkylated or alkenylated as mentioned above, the foregoing compound 5 or 6 gives the corresponding alkylated or aleknylated α-fluoro-α-cyanoacetic acid or its alkali metal salt.

If subjected to the Michael reaction as mentioned above, the same compound 5 or 6 gives the corresponding Michael adduct of α-fluoro-α-cyanoacetic acid or its alkali metal salt.

The invention will be understood more clearly with reference to the following Examples:

Example 1

In a three-necked flask equipped with an efficient dry-ice condenser, placed 1000 g of conc. ammonia water and 500 ml of dioxane. Into this mixture, 291 g of hexafluoro propene gas was introduced at the rate of 0.9 g/min, keeping the temperature at $0 \pm 2$ °c . After the introduction, the reaction mixture was stirred for 2 hrs at -5 to 0 °c, extracted with xylene, washed with water, dried over magnesium sulfate and subjected to distillation.

Thus 183 g of 2,3,3,3-tetrafluoropropio-nitrile, b.p. 39-40 °c, was obtained in 74 % yield.

Instead of 1,4-dioxane in the preceding procedure, the same amount of tetrahydrofuran was used.

In a similar manner 295 g of hexafluoropropene was made react, giving 206 g (82 % yield) of 2,3,3,3-tetrafluoropropionitril.

In a 21-flask placed a solution of 120 g (3 mol) of sodium hydroxide in 11 of ethanol, and 127 g (1 mol) of 2,3,3,3-tetrafluoropropionitrile was added dropwise at room temperature and stirred for 1 hr. From the reaction mixture 500 ml of ethanol distilled out and the residue was poured into 11 of water. Oily material was extracted with chloroform, washed with water 5 times, and dried over calcium chloride.

Into the extract thus obtained was added 80 ml of conc. hydrochloric acid and the mixture was stirred at room temperature for 1h. After neutralization with a saturated aqueous solution of sodium bicarbonate, an oily layer was separated, washed with water and dried over magnesium sulfate. On removing chloroform 79 g (60 %) of ethyl α-fluoro-α-cyanoacetate and 46 g (26 %) of ethyl fluoromalonate were distilled out at 174-175 °c and 208-210 °c, respectively. The product, that is, ethyl α-fluoro-α-cyanoacetate showed the following properties:

| | |
|---|---|
| IR(neat): | 2260 (CN), 1780,1760 $cm^{-1}$ (Ester) |
| $^{19}F$ NMR($CHCl_3$): | $\delta$ 114.0 (d) ($J_{HF}$=45.5 Hz) |
| $^{1}H$ NMR($CDCl_3$): | $\delta$ 5.47 (d) ($J_{HF}$=47.4 Hz), 4.30(q), 1.30 (t) |
| MS (m/e): | 131 ($M^+$) |

Exampl 2

Into a mixture of 290 mg (5 mmol) of spray-dried potassium fluoride and 10 ml of sulforane, 2.65 g (20 mmol) of ethyl α-fluoro-α-cyano acetate was added dropwise at room temperature. After agitation of the mixture for 10 minutes, the reaction solution was added dropwise by 2.92 g (20 mmol) of benzal acetone under ice cooling, then stirred for 10 minutes at 0 to 5 °c. Thereafter, a saturated ammonium chloride solution was added to the mixture, an oily material was extracted with ether and dried over magnesium sulfate. After the ether was distilled out, 4.7 g of a Michael adduct of b.p. 141 to 142 °c/2mmHg was produced in a yield of 85 % by a reduced-pressure distillation. This product showed the following analytical properties:

| | |
|---|---|
| IR (neat): | 2260 (CN), 1715 (CO), 1770 $cm^{-1}$ (Ester) |
| $^{19}F$ NMR ($CCl_4$): | δ 78.9 (d, $J_{HF}$=22.0 Hz), 81.4 (d, $J_{HF}$ =22.9 Hz) |
| $^{1}H$ NMR ($CCl_4$): | δ 1.00-1.30 (2t), 2.00 (s), 3.05 to 3.21 (2d), 3.80-4.32 (2q+m), 7.22-7.48 (m) |
| MS (m/e): | 277 ($M^+$) |

Example 3

Into a mixture of 290 mg (5 mmol) of spray-dried potassium fluoride and 10 ml of sulforane, 2.65 g (20 mmol)

of ethyl α-fluoro-α-cyanoacetate was added dropwise at room temperature. After agitation for 10 minutes at 0 to 5 °c, the reaction mixture was added dropwise by 3.25 g (20 mmol) of methyl cinnamate under ice cooling and these were reacted for 15 hours at room temperature. Then, the reaction mixture was treated similarly to Example 2 to produce 4.8 g of a Michael adduct of b.p. 134-135 °c/3 mmHg in a yield of 83 % through a reduced-pressure distillation. This product had the following properties:

IR (neat): 2260 (CN), 1770, 1745 $cm^{-1}$ (Ester)

$^{19}F$ NMR ($CCl_4$): δ 79.5 (d, $J_{HF}$=20.7 Hz),

83.5 (d, $J_{HF}$=23.5 Hz)

MS (m/e): 293 ($M^+$)

Example 4

Into a mixture of 480 mg (20 mmol) of NaH and 10 ml of diglyme, 2.65 g (20 mmol) of ethyl α-fluoro-α-cyanoacetate in 5 ml of diglyme was added dropwise under ice cooling. After agitation for 20 minutes, the reaction mixture was added dropwise by 3.32 g (22 mmol) of methyl iodide at the same temperature and these were reacted for 2 hours at room temperature. The resulted reaction mixture was added by a saturated aqueous ammonium chloride solution, then an oily material was extracted with ether, washed with water, dried over magnesium sulfate. After distilling-out of the ether, a reduced-pressure distillation resulted in 1.0 g of a methylated product of b.p. 91-93 °c/20 mmHg

in a yield of 71 %. The analytical data of this product were as follows:

IR (neat): 2260 (CN), 1780 $cm^{-1}$ (Ester)

$^{19}F$ NMR ($CCl_4$): $\delta$ 71.0 (q, $J_{HF}$=21.8 Hz)

$^{1}H$ NMR ($CCl_4$): $\delta$ 1.32 (t), 1.72 (d), 4.32(t)

MS (m/e): 145 ($M^+$)

WHAT IS CHAIMED IS:

1. An α-substituted-α-fluoro-α-cyanoacetic acid and its derivatives that are expressed by a general formula:

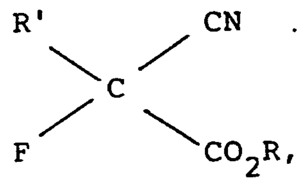

where R is an aliphatic or aromatic hydrocarbon group, or hydrogen or alkali metal atom, and R' is an aliphatic or aromatic hydrocarbon group or a group that derived from an acceptor of the Michael reaction.

2. An α-substituted-α-fluoro-α-cyanoacetic acid and its derivatives as claimed in claim 1 wherein the R is an alkyl or alkenyl group haveing up to 10 carbon atoms, or aryl group.

3. An α-substituted-α-fluoro-α-cyanoacetic acid and its derivatives as claimed in claim 1 or 2 wherein the R' is an alkyl or alkenyl group having up to 10 carbon atoms, or aryl group.

4. An α-substituted-α-fluoro-α-cyanoacetic acid and its derivatives as claimed in claim 3 wherein the group R' is a group as derived from an unsaturated compound expressed by the following general formula:

YCH=CHR" or YC≡CR",

where R" is a hydrogen atom or aliphatic group and Y is a highly electronegative group with or without formation of a

ring by bonding to a carbon atom to which the group R" is bonded.

5. An α-substituted-α-fluoro-α-cyanoacetic acid and its derivatives as claimed in claim 4 wherein the group Y is an alkylcarbonyl or alkoxycarbonyl or cyano group bonded to a carbon atom comprising the unsaturated carbon-carbon bond.